# EUROPEAN PATENT APPLICATION

(11) **EP 4 831 162 A1**
(43) Date of publication of application: **28.10.2026**
(21) Application number: 24908397.3
(22) Date of filing: 25.09.2024
(51) Int. Cl.: C07D 261/04, A61K 9/06, A61K 31/42, A61K 47/38, A01N 43/80

(54) **ORAL COMPOSITION IN BIOADHESIVE GEL FORM BASED ON ISOXAZOLINE**

(30) Priority: 21.12.2023 PE 0034432023
(71) Applicant: Agrovet Market S.A., San Luis, Lima, 15019 (PE)
(72) Inventor: CALDERON OJEDA, Jorge Umberto, Lima, 15024 (PE); DELGADO CRISPIN, Karen Melissa, Lima, 15043 (PE); MOLLEAPAZA AGUILAR, Erick Alfredo, Lima, 15246 (PE); PORRAS NICHO, Mauricio Yvan, Lima, 15096 (PE)
(74) Representative: JD&P Patent Attorneys Joanna Dargiewicz & Partners
(86) International application number: PCT/PE2024/050020
(87) International publication number: WO 2025/136126

(57) **Abstract**

The present invention relates to a composition of a long-acting bioadhesive endoparasiticide gel based on an isoxazoline alone or in combination with other antiparasitic agents, having a high degree of adhesion to oral mucosa and high palatability greatly facilitating the dosing thereof, and to methods for preparing the same. Ectoparasitosis is a parasitic disease caused by fleas, ticks and mites that infest the surface layers of the skin in small animals. Control of this illness is of great importance and advantageously achievable through the use of appropriate antiparasitic agents such as isoxazolines, being a chemical compound widely administered by oral route in tablet form due to the organoleptic and physicochemical characteristics of the compositions known up to the present. The present invention demonstrates that it is possible to administer isoxazolines in the pharmaceutical form of an oral gel at concentrations of up to 20.0% weight/volume, overcoming the organoleptic and physicochemical restrictions of the compositions known up to the present. Factors never heretofore included in oral endoparasiticide compositions based on isoxazolines, presenting a wide differential advantage compared with the compositions known up to the present.

## Description

### TECHNICAL FIELD

The present invention pertains to the technical field of the pharmaceutical industry, mainly to the industry of pharmaceutical products for veterinary use.

### PRIOR ART

The development of pharmaceutical compositions or antiparasitic formulations that have activity against external parasitosis caused by fleas, ticks, and mites and that are easy to administer is a current necessity for the treatment and control of infestations in small animals.

In the prior art, high penetration compositions or prodrugs of antimicrobials and antimicrobial related compounds are known, such as CN 105566213 which discloses compositions capable of converting into original drugs or drug metabolites after crossing the biological barrier and, therefore, can offer treatments. In addition, the compositions are able to reach areas that their original drugs may not be able to access and generate a sufficient concentration in the target areas and, therefore, deliver innovative treatments. Formulations suitable for oral administration may be capsules, seals, pills, lozenges (using astringent-based ingredients, typically sucrose, acacia or tragacanth), powders, granules or a solution or suspension in an aqueous or non-aqueous liquid, or a liquid emulsion of oil in water or water in oil, or an elixir or syrup, or a tablet (using an inert substrate, e.g., gel and glycerin, or sucrose and gum arabic) and/or mouth rinses and the like, each of which contains a predetermined amount of active ingredients such as antibiotics derived from isoxazoline among others. The compound can also be administered as a bolus, electrolyte, or paste.

In turn, document ES2182485 discloses new substituted aminophenyl isoxazoline derivatives and pharmaceutical compositions containing them as active ingredients and procedures for their use. Compounds of the invention are useful as antimicrobial agents for preventing and treating infectious diseases in humans or animals infected with pathogens, with oral administration being the preferred form. Pharmaceutical compositions for parenteral administration will generally contain a pharmaceutically acceptable amount of the compounds according to formula 1 as a soluble salt (acid addition salt or base salt) dissolved in a pharmaceutically acceptable liquid vehicle and be dissolved in the vehicle in an amount sufficient to provide a pharmaceutically acceptable concentration are administered orally in solid and liquid dosage forms comprising gelatin, cellulosic materials low melting point waxes among others.

Also known is document US2021/0220360 which discloses a hard palatable chewable composition comprising at least one veterinarily acceptable isoxazoline, a stabilized macrocyclic lactone, an acceptable salt form of pyrantel, at least one natural palatant of animal origin, and at least one veterinarily acceptable excipient. The invention also contemplates a method of use for treating and/or preventing a parasitic infection or infestation in an animal in need thereof by administering said composition to the animal, wherein the composition comprises binders to add cohesiveness to the separated granulations and the final mixed composition, thereby providing the necessary binding to form a cohesive mass and ensure a compacted tablet form and veterinary acceptable binders include, microcrystalline cellulose, carboxymethylcellulose, sodium carboxymethyl cellulose, hydroxypropyl cellulose (HPC), polyvinylpyrrolidone and copovidone, polyethylene glycol, acacia, corn syrup solids, gum tragacanth, gelatin, carnauba wax, alginate and mixtures thereof.

In turn, document WO2022/212399 provides methods for treating a clinical symptom and/or transmission risk associated with a pathogen in an individual, including related pharmaceutical formulations, the method comprising the steps of: administering to the individual having the clinical symptom or at risk of transmission of the pathogen, an active agent in an effective dose to inactivate Demodex mites in or on the individual; thereby resulting in an improvement or cessation of clinical symptoms and/or the risk of transmission associated with the pathogen, wherein the active ingredient may be applied in conjunction with pharmaceutically acceptable carriers such as lotions, creams, soaps, shampoos and gels.

In this regard, there is still a need to provide an oral pharmaceutical composition or formulation that in the form of a bioadhesive gel comprising as active ingredient an isoxazoline that may be alone or in combination with other antiparasitics and that is easy to administer and adhere to the product should be applied to the mouth or to the inner walls of the mouth or to the palate of the animal in such a way as to ensure that the drug can be fully dosed to provide comprehensive protection against external parasites such as fleas, ticks and mites. The proposed composition contains an active ingredient of the isoxazoline family, which is highly effective against parasitosis and also allows for multi-dose administration of the drug.

The proposed composition is a broad-spectrum parasiticide containing an isoxazoline alone or in combination with other antiparasitics, available in gel form with bioadhesive characteristics that achieves that the pharmaceutical composition for veterinary use has a high palatability, since the adhesiveness of the gel provides a greater ease in the drug delivery system allows the product to adhere in the animal's mouth and ensures that the correct dosage is delivered. The composition of oral administration achieves a good adhesion in the oral cavity and its use in the prevention and/or treatment of infestations of domestic animals for external parasites.

### BRIEF DESCRIPTION OF THE INVENTION

The present invention is directed to a formulation or pharmaceutical composition and a method of manufacturing or producing the same, wherein the pharmaceutical composition comprises an isoxazoline alone or in combination with other antiparasitics for the treatment of parasitic infestations in animals, wherein the composition is in the form of a bioadhesive oral gel.

### DETAILED DESCRIPTION OF THE INVENTION

The present invention relates to the composition of an isoxazoline-based endoparasiticidal bioadhesive gel with a high level of adhesion to the oral mucosa of treated animals treated and good palatability that greatly facilitates its dosage and preparation procedures.

External parasitosis caused by fleas, ticks and mites is a very frequent affectation in companion animals, due to this an effective control of parasitosis is of great importance, which can be achieved with antiparasitic drugs of proven efficacy such as isoxazolines, but they must be easy to administer to achieve it.

The present invention refers to a composition or pharmaceutical formulation for oral administration of an isoxazoline for the treatment of parasitic infestations in small animals, wherein the composition comprises as isoxazoline or a salt or solvate of isoxazoline having as its structure of formula 1:

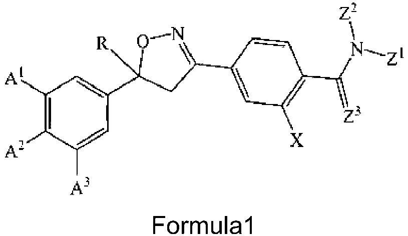

An isoxazoline, salt or solvate according to the structure of formula 1 wherein A1, A2 and A3 are selected from the group consisting of hydrogen, halogen and halomethyl; and wherein R is a halomethyl; and wherein X is selected from the group consisting of hydrogen, halogen, methyl, halomethyl, ethyl and haloethyl; and where Z1 and Z2 are substituents selected from the group consisting of hydrogen, methyl, haloethyl, halopropyl, halobutyl, methoxymethyl, halomethoxymethyl, ethoxymethyl, haloethoxymethyl, propoxymethyl, ethylaminocarbonylmethyl, ethylaminocarbonylmethyl, dimethoxyethyl, propinylaminocarbonylmethyl, N-phenyl-N-methyl-amino, haloethylaminocarbonylmethyl, haloethylaminocarbonylethyl. tetrahydrofuryl, methylaminocarbonylmethyl, (N,N-dimethylamino)-carbonylmethyl, propylaminocarbonylmethyl, cyclopropylaminocarbonylmethyl, propenylaminocarbonylmethyl, haloethylaminocarbonylcyclopropyl; and wherein Z3 consists of O and S.

An isoxazoline, salt or solvate according to the structure of formula 1, wherein A1, A2 and A3 is halogen and may be fluoro, bromo or chloro.

An isoxazoline, salt or solvate according to the structure of formula 1, where A1 and A2 is halomethyl and is trifluoromethyl.

An isoxazoline, salt or solvate according to the structure of formula1, wherein R is monochloromethyl, trifluoromethyl, monochloro-difluoromethyl.

An isoxazoline, salt or solvate according to the structure of formula 1, where X is hydrogen, bromine, iodine, chlorine, methyl, ethyl, trifluoromethyl.

An isoxazoline, salt or solvate according to the structure of formula 1, wherein Z1 and Z2 are selected from the group consisting of hydrogen, methyl, haloethyl, halopropyl, halobutyl, methoxymethyl, halomethoxymethyl, ethoxymethyl, haloethoxymethyl, propoxymethyl, ethylaminocarbonylmethyl, ethylaminocarbonylethyl, dimethoxyethyl, propynylaminocarbonylmethyl, N-phenyl-N-methyl-amino, haloethylaminocarbonylmethyl, haloethylaminocarbonylethyl. tetrahydrofuryl, methylaminocarbonylmethyl, (N,N-dimethylamino)carbonylmethyl, propylaminocarbonylmethyl, cyclopropylaminocarbonylmethyl, propenylaminocarbonylmethyl, haloethylaminocarbonylcyclopropyl; and wherein Z3 consists of oxygen or sulfur.

The isoxazoline used in the present invention may have two or more conformational structures, but at a minimum comprise one chiral carbon at position 5 of the isoxazoline ring. Other isoxazolines and their salts or solvates comprised in the present invention, are selected from fluralaner, sarolaner, afoxolaner and lotinaler or combinations thereof and may be in combination with other antiparasitics.

Surprisingly isoxazolines have never been used under the pharmaceutical form of oral gel. This chemical compound is very frequently administered orally due to its organoleptic and physicochemical characteristics of the compositions known to date in tablet form.

The present invention favorably demonstrates, unlike the aforementioned patents, that it is possible to administer an isoxazoline under the pharmaceutical form of oral gel at concentrations of 0.01% to 30.00%, long-acting, highly palatable and of great adhesion to the oral cavity. Factors not included in isoxazoline-based oral antiparasitic compositions that present a broad differential advantage over compositions known to date.

For such reason, the present invention has as its objectives to provide:
- A parasiticidal oral gel composition based on isoxazoline for small animals.
- A parasiticidal oral gel composition based on isoxazoline in concentrations of up to 30% of said active principle alone or in combination with other antiparasitics.
- A long-acting isoxazoline-based antiparasitic oral gel composition with a proven effectiveness of at least 60 days after administration.
- An oral antiparasitic gel composition based on isoxazoline with a high palatability to encourage voluntary ingestion.
- An antiparasitic oral gel composition with a high degree of adhesion to the mucous membranes of the oral cavity that reduces the likelihood of voluntary or involuntary expulsion of the gel after administration.

This bioadhesive oral antiparasitic gel composition comprises:
a) From 0.10% to 30.00%; preferably from 0.50% to 25.00%; more preferably from 1.00% to 20.0% wt/vol of an isoxazoline.
b) From 2.00% to 10.00% wt/vol of a viscous agent such as cellulose, ethyl cellulose, methyl cellulose, silicon dioxide colloidal, hydroxypropyl methylcellulose, povidone, similar components, or mixtures thereof.
c) From 1.00% to 80.00%; preferably from 5.00% to 70.00%; more preferably from 10.00% to 60.00% weight/volume of a diluent suitable as propylene glycol, polyethylene glycol 300, polyethylene glycol 400, glycerin, water, similar components, or mixtures thereof.
d) From 1.00% to 40.00%; preferably from 2.00% to 30.00%; more preferably from 2.50% to 20.00% wt/vol of a solvent such as N-methyl-2-pyrrolidone, 2-pyrrolidone, dimethyl sulfoxide, propylene glycol, water, benzyl alcohol, glycerin, formal glycerol, vegetable oil which may be soybean oil, coconut oil, sesame oil, corn oil, among others; polyethoxylated castor oil, salmon oil, macrogol 15 hydroxystearate similar components or mixtures thereof.
e) From 1.00% to 50.00%; preferably from 2.00% to 40.00%; more preferably from 5.00% to 35.00% wt/vol of a natural or synthetic flavoring agent.
f) From 0.01% to 4.00%; preferably from 0.05% to 3.00%; more preferably from 0.10% to 2.50% by volume weight of a flavoring agent which is a natural or synthetic essence.
g) From 0.01% to 5.00%; preferably from 0.05% to 3.00%; more preferably from 0.10% to 2.50% weight by volume of a sweetener selected from sucralose, saccharin, aspartame, cyclamate, similar components, or mixtures thereof.
h) From 0.01% to 1.00%; more preferably from 0.05% to 0.5% wt/vol of a preservative agent such as methylparaben, propylparaben, butylparaben, similar compounds or mixtures thereof.
i) From 0.01% to 1.00%; more preferably from 0.02% to 0.5% wt/vol of an antioxidant agent are advantageously chosen from among ethyl or propyl gallate , alpha tocopherol, ascorbic acid, ascorbyl palmitate, monothioglycerol, butylated hydroxytoluene (BHT) and butylated hydroxyanisole (BHA); as well as mixtures thereof.

The composition according to the present invention, may further comprise another antiparasitic in combination, which may be, but is not limited to, for example, a pyrazinoisoquinoline such as praziquantel, pyrantel, febantel or combinations thereof.

The composition according to the present invention, may comprise an antiparasitic in combination is a macrocyclic lactone, selected from an avermectin from the group consisting of ivermectin, emamectin, eprinomectin, selamectin, doramectin, moxidectin, abamectin or combinations thereof.

In a second aspect, the present invention refers to the method of producing a pharmaceutical composition comprising an isoxazoline, for the treatment of parasitic infestations in animals minor, wherein the method comprises the following steps:
a) Mixing isoxazoline and selected flavorings and sweeteners;
b) Mixing the selected antioxidants and the flavoring agent with the selected solvent(s), stir until complete dissolution until solution A is formed;
c) Adding the selected preservatives to solution A and stir until solution B is formed;
d) Adding the diluent or diluents to solution B, stir until forming solution C;
e) On the solution C, incorporating the selected viscous agent, until a homogeneous dispersion is formed, the suspension D is formed; and
f) Mixing suspension D with solution A and the mixture from the first step.
g) Bringing to the final volume with the selected diluent

In a third aspect, the invention further comprises a method of treating an animal, wherein said method is for treating a parasitic infestation in an animal in need thereof, comprising administering to said animal a bioadhesive oral gel composition comprising as active ingredient an isoxazoline which may be alone or in combination with other antiparasitics.

In a fourth aspect, the invention further comprises use of a bioadhesive oral gel composition comprising as an active ingredient an isoxazoline that may be alone or in combination with other antiparasitics for the treatment of an animal in need of such treatment.

### Examples of formulation or compositions according to the invention

Examples of the declared composition are shown below in Table N°1.

**Table N°1. Examples of formulas with declared composition**

| Component | A | B | C | D | E | F |
|---|---|---|---|---|---|---|
| Fluralaner | 7.500% | 7.500% | 7.500% | 7.500% | 7.500% | 7.500% |
| Microcrystalline cellulose | 7.500% | 0.000% | 0.000% | 0.000% | 0.000% | 0.000% |
| Hydroxypropylmethylcellulose | 1.000% | 0.000% | 0.000% | 0.000% | 1.000% | 2.500% |
| Carboxymethylcellulose | 0.000% | 1.500% | 0.000% | 0.000% | 0.000% | 0.000% |
| Silicon dioxide | 0.000% | 0.000% | 5.000% | 6.000% | 6.000% | 1.500% |
| Glycerin | 0.000% | 30.000% | 0.000% | 0.000% | 0.000% | 49.260% |
| Propylene glycol | 0.000% | 28.260% | 20.000% | 0.000% | 0.000% | 2.500% |
| Ethyl alcohol | 3.000% | 0.000% | 0.000% | 0.000% | 0.000% | 0.000% |
| Dimethyl sulfoxide | 0.000% | 11.000% | 7.600% | 8.000% | 8.000% | 0.000% |
| Purified water | 0.000% | 20.000% | 0.000% | 0.000% | 0.000% | 0.000% |
| Vegetable oil | 25.000% | 0.000% | 25.000% | 20.000% | 20.000% | 0.000% |
| Salmon oil | 46.760% | 0.000% | 30.160% | 38.160% | 37.160% | 10.000% |
| Flavoring | 8.000% | 1.000% | 4.000% | 20.000% | 20.000% | 25.000% |
| Sucralose | 0.000% | 0.000% | 0.000% | 0.000% | 0.000% | 1.000% |
| Aroma | 1.000% | 0.500% | 0.500% | 0.100% | 0.100% | 1.000% |
| Butyl hydroxy toluene | 0.020% | 0.020% | 0.020% | 0.020% | 0.020% | 0.020% |
| Butyl hydroxy anisole | 0.020% | 0.020% | 0.020% | 0.020% | 0.020% | 0.020% |
| Methyl paraben | 0.180% | 0.180% | 0.180% | 0.180% | 0.180% | 0.180% |
| Propyl paraben | 0.020% | 0.020% | 0.020% | 0.020% | 0.020% | 0.020% |
| Total: | 100.000% | 100.000% | 100.000% | 100.000% | 100.00% | 100.00% |

### Palatability study of the composition of the present invention

### Study design

For the palatability test, 45 canines, older than 8 weeks of age, of both sexes, of different weights, of various breeds and clinically healthy, were used.

The dosing syringe was presented at muzzle height, and a score for the product was determined according to the reaction based on the scale shown in Table N°2.

**Table N°2 Palatability scale in canines.**

| **Score** | **Description** |
|---|---|
| 0 | The animal must be forced to eat the contents of the dosing syringe |
| 1 | The animal shows interest at first, but the contents of the dosing syringe must be forced |
| 2 | The animal shows interest and eats the content of the dosing syringe when it is placed on the snout |
| 3 | The animal sniffs the contents of the dosing syringe and eats it voluntarily |

### Results

Table 3 shows the average, as well as the percentage of voluntary acceptance and rejection for each sample evaluated.

Gel consistency is a determining point in product acceptance. Samples with a high adhesiveness to the oral cavity such as samples D and E have a higher percentage of acceptance.

**Table N°3. Palatability test results**

| Sample | A | B | C | D | E | F |
|---|---|---|---|---|---|---|
| Average | 1.5 | 0.65 | 0.75 | 2.0 | 2.4 | 2.80 |
| Rejection | 25% | 50% | 65% | 15% | 10% | 0% |
| Acceptance | 75% | 50% | 35% | 85% | 90% | 100% |

## Claims

1. A bioadhesive oral gel composition for treatment of parasitic infestations in small animals is **characterized in that** it comprises as active ingredient an isoxazoline that can be alone or in combination with other antiparasitics.

2. The composition according to claim 1, wherein the isoxazoline compound belongs to the chemical structure of formula 1:

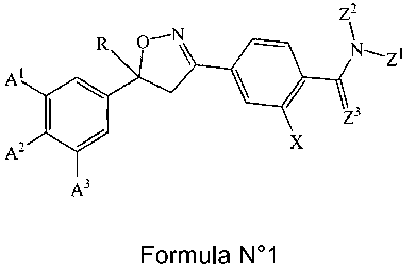

3. The compound according to claim 2, wherein A1, A2 and A3 are selected from the group consisting of hydrogen, halogen and halomethyl; and wherein R is a halomethyl; and wherein X is selected from the group consisting of hydrogen, halogen, methyl, halomethyl, ethyl and haloethyl; and wherein Z1 and Z2 are substituents selected from the group consisting of hydrogen, methyl, haloethyl, halopropyl, halobutyl, methoxymethyl, halomethoxymethyl, ethoxymethyl, haloethoxymethyl, propoxymethyl, ethylaminocarbonylmethyl, ethylaminocarbonylethyl, dimethoxyethyl, propynylaminocarbonylmethyl, N-phenyl-N-methyl-amino, haloethylaminocarbonylmethyl, haloethylaminocarbonylethyl. tetrahydrofuryl, methylaminocarbonylmethyl, (N,N-dimethylamino)-carbonylmethyl, propylaminocarbonylmethyl, cyclopropylaminocarbonylmethyl, propenylaminocarbonylmethyl, haloethylaminocarbonylcyclopropyl; and wherein Z3 consists of O and S.

4. The composition according to claim 2, wherein A1, A2 and A3 is halogen and may be fluorine, bromine, or chlorine.

5. The composition according to claim 2, wherein A1 and A2 are halomethyl and trifluoromethyl.

6. The composition according to claim 2, wherein R is monochloromethyl, trifluoromethyl, monochloro-difluoromethyl.

7. The composition according to claim 2, wherein X is hydrogen, bromine, iodine, chlorine, methyl, ethyl, trifluoromethyl.

8. The composition according to claim 2, wherein Z1 and Z2 are selected from the group consisting of hydrogen, methyl, haloethyl, halopropyl, halobutyl, methoxymethyl, halomethoxymethyl, ethoxymethyl, haloethoxymethyl, propoxymethyl, ethylaminocarbonylmethyl, ethylaminocarbonylethyl, dimethoxyethyl, propinylaminocarbonylmethyl, N-phenyl-N-methyl-amino, haloethylaminocarbonylmethyl, haloethylaminocarbonylmethyl, tetrahydrofuryl, methylaminocarbonylmethyl, (N,N-dimethyl-amino)carbonylmethyl, propylaminocarbonylmethyl, cyclopropylaminocarbonylmethyl, propenylaminocarbonylmethyl, haloethylaminocarbonylcyclopropyl; and wherein Z3 consists of oxygen or sulfur.

9. The composition according to claim 1, wherein the compound of isoxazoline, its salts or solvates are selected from fluralaner, sarolaner, afoxolaner and lotinaler or combinations thereof.

10. The composition according to claim 1, wherein the antiparasitic in combination may comprise a macrocyclic lactone selected from the group consisting of ivermectin, emamectin, eprinomectin, selamectin, doramectin, moxidectin, abamectin or combinations thereof.

11. The composition according to claim 1, wherein the antiparasitic in combination may comprise praziquantel, pyrantel, febantel or combinations thereof.

12. The composition according to claim 1, wherein the active ingredients may be present in a concentration between 0.1 and 30% based on the total weight of the composition; preferably from 0.50% to 25.00%; more preferably from 1.00% to 20.0% wt/vol of an isoxazoline.

13. The composition according to claim 1, wherein a solvent such as N-methyl-2-pyrrolidone, 2-pyrrolidone, dimethyl sulfoxide, propylene glycol, water, benzyl alcohol, glycerin, formal glycerol, vegetable oil such as soybean oil, coconut oil, sesame oil, corn oil, among others; polyethoxylated castor oil, salmon oil, macrogol hydroxystearate, similar components or mixtures thereof may be present in a concentration from 1.00% to 40.00%; preferably from 2.00% to 30.00%; more preferably from 2.50% to 20.00% wt/vol.

14. The composition according to claim 1, wherein a viscous agent such as cellulose, ethyl cellulose, methyl cellulose, colloidal silicon dioxide, hydroxypropyl methylcellulose, povidone, similar components, or mixtures thereof may be present in a concentration of 2.00% to 10.00% wt/vol.

15. The composition according to claim 1, wherein a suitable diluent as propylene glycol, polyethylene glycol 300, polyethylene glycol 400, glycerin, water, similar components, or mixtures thereof may be present in a concentration of from 1.00% to 80.00%; preferably from 5.00% to 70.00%; more preferably from 10.00% to 60.00% weight/volume.

16. The composition according to claim 1, wherein a natural or synthetic flavoring agent may be present in a concentration of from 1.00% to 50.00%; preferably from 2.00% to 40.00%; more preferably from 5.00% to 35.00% weight/volume.

17. The composition according to claim 1, wherein a flavoring agent which is a natural or synthetic essence may be present in a concentration of from 0.01% to 4.00%; preferably from 0.05% to 3.00%; more preferably from 0.10% to 2.50% weight by volume.

18. The composition according to claim 1, wherein a sweetening agent selected from sucralose, saccharin, aspartame, cyclamate, similar components, or mixtures thereof may be present in a concentration of from 0.01% to 5.00%; preferably from 0.05% to 3.00%; more preferably from 0.10% to 2.50% weight by volume.

19. The composition according to claim 1, wherein a preservative agent such as methylparaben, propylparaben, butylparaben, butylparaben, similar components or mixtures thereof may be present in a concentration of from 0.01% to 1.00%; more preferably from 0.05% to 0.5% weight/volume.

20. The composition according to claim 1, wherein an antioxidant agent such as ethyl or propyl gallate, alpha tocopherol, ascorbic acid, ascorbyl palmitate, monothioglycerol, butylated hydroxytoluene (BHT) and butylated hydroxyanisole (BHA); as well as mixtures thereof can be present in a concentration of 0.01% to 1.00%; more preferably from 0.02% to 0.5% wt/volume.

21. A method of preparing a composition according to claim 1,
comprising the following stages:
a) Mixing the isooxazoline and the flavorings and the selected sweeteners;
b) Mixing the selected antioxidants and the flavoring agent with the selected solvents, stirring until complete dissolution until solution A is formed;
c) Adding the selected preservatives to solution A and stir until solution B is formed;
d) Adding the diluent(s) to solution B, stir until the solution C is formed;
e) Adding the selected viscous agent to the solution C , until a homogeneous dispersion is formed, the suspension D is formed; and.
f) Mixing suspension D with solution A and the mixture from the first step.

22. A method for treating a parasitic infestation in an animal in need thereof, comprising administering to said animal an oral bioadhesive gel composition, comprising administering to said animal a bioadhesive oral gel composition comprising as an active ingredient an isoxazoline, which may be alone or in combination with other antiparasitics.

23. The use of a bioadhesive oral gel composition comprising as an active ingredient an isoxazoline, which may be alone or in combination with other antiparasitics, for the treatment of an animal in need of such treatment for a parasitic infestation
